# EUROPEAN PATENT SPECIFICATION

(11) **EP 4 244 375 B1**
(45) Date of publication and mention of the grant of the patent: **17.12.2025**
(21) Application number: 21892573.3
(22) Date of filing: 29.10.2021
(51) Int. Cl.: C12Q 1/48, G01N 33/564, G01N 33/50, G01N 33/573, A61P 37/06, G01N 33/68

(54) **METHODS AND MATERIALS FOR IDENTIFYING AND TREATING MEMBRANOUS NEPHROPATHY**
VERFAHREN UND MATERIALIEN ZUR IDENTIFIZIERUNG UND BEHANDLUNG VON MEMBRANÖSER NEPHROPATHIE
PROCÉDÉS ET SUBSTANCES POUR IDENTIFIER ET TRAITER UNE NÉPHROPATHIE MEMBRANEUSE

(30) Priority: 10.11.2020 US 202063111982 P
(43) Date of publication of application: 20.09.2023
(73) Proprietor: Mayo Foundation for Medical Education and Research, Rochester, Minnesota 55905 (US)
(72) Inventor: FERVENZA, Fernando C., Rochester, Minnesota 55902 (US); SETHI, Sanjeev, Rochester, Minnesota 55902-1335 (US)
(74) Representative: Fish & Richardson P.C.
(86) International application number: PCT/US2021/057259
(87) International publication number: WO 2022/103598

(56) References cited:
- WO-A1-2020/037135
- US-A1- 2018 203 020
- RONCO PIERRE ET AL: "Membranous nephropathy", NATURE REVIEWS DISEASE PRIMERS, NATURE PUBLISHING GROUP UK, LONDON, vol. 7, no. 1, 30 September 2021 (2021-09-30), XP037577265, DOI: 10.1038/S41572-021-00303-Z
- SETHI ET AL.: "Exostosin 1/Exostosin 2-Associated Membranous Nephropathy", J AM SOC NEPHROL, vol. 30, no. 6, June 2019 (2019-06-01), pages 1123 - 1136, XP055806004, DOI: 10.1681/ASN.2018080852
- RAVINDRAN ET AL.: "In Patients with Membranous Lupus Nephritis, Exostosin-Positivity and Exostosin-Negativity Represent Two Different Phenotypes", J AM SOC NEPHROL, vol. 32, no. 3, March 2021 (2021-03-01), pages 695 - 706, XP055806009, DOI: 10.1681/ASN.2020081181

## Description

### CROSS-REFERENCE TO RELATED APPLICATIONS

This application claims the benefit of U.S. Patent Application Serial No. 63/111,982, filed on November 10, 2020.

### BACKGROUND

### 1. Technical Field

This document relates to methods and materials involved in identifying mammals having membranous nephropathy (e.g., lupus membranous nephritis) likely or unlikely to progress to end stage kidney disease. For example, this document provides methods and materials for determining if a mammal having membranous nephropathy (e.g., lupus membranous nephritis) is likely or unlikely to progress to end stage kidney disease. This document also provides methods and materials for administering one or more immunosuppressive agents (e.g., corticosteroids, cyclosporine, mycophenolate mofetil, or a B-cell reduction or depletion agent such as Rituximab) to a mammal (e.g., a human) having membranous nephropathy that was identified as being likely to progress to end stage kidney disease.

### 2. Background Information

Membranous nephropathy (MN) is the most common cause of nephrotic syndrome in white adults (Couser, Clin. J. Am. Soc. Nephrol., 12:983-997 (2017); Makker et al., Semin. Nephrol., 31:333-340 (2011); and Ronco et al., The Lancet, 385:1983-1992 (2015)). Primary MN accounts for approximately 70-80% of cases, and the remaining 20-30% cases are secondary MN. In the primary MN, M-Type Phospholipase A2 Receptor antigen (PLA2R), Thrombospondin Type-1 Domain-Containing 7A antigen (THSD7A), Neural epidermal growth factor-like 1 protein (NELL1), and Semaphorin 3B (Sema3B) have been identified as target antigens (Beck et al., N. Engl. J. Med., 361:11-21 (2009); Tomas et al., N. Engl. J. Med., 371:2277-2287 (2014); Sethi et al., Kidney International, 97:163-174 (2020); and Sethi et al., Kidney International, 98:5-1253-1264 (2020)). Secondary MN is associated with autoimmune diseases, malignancies, infection, and drugs (Couser, Clin. J. Am. Soc. Nephrol., 12:983-997 (2017)). Recently, two novel proteins, Exostosin 1 (EXT1) and Exostosin 2 (EXT2), were recently discovered in the glomerular basement membrane (GBM) in a small cohort of PLA2R-negative MN (Anders et al., Nature Rev. Nephrol., 15:595-596 (2019); and Sethi et al., J. Am. Soc. Nephrol., 30:1123-1136 (2019)). More than 80% of EXT1/EXT2-positive patients had an autoimmune finding, confirmed by serology, clinical presentation, or kidney biopsy findings of an underlying autoimmune disease (Sethi et al., J. Am. Soc. Nephrol., 30:1123-1136 (2019)).

WO 2020/03175 A1 describes methods for identifying and treating membranous nephropathy which comprise detecting the presence of autoantibodies specific for EXT1 and/or EXT2 polypeptides or kidney tissue having an elevated level of an EXT1 and/or EXT2 polypeptide.

SETHI et al.: "Exostosin 1/Exostosin 2-Associated Membranous Nephropathy", J Am Soc Nephrol, vol. 30, no. 6, June 2019 (2019-06), pages 1123-1136, DOI: 10.1681/ASN.2018080852 discloses a method of identifying a patient with membranous nephropathy (MN) but the progression of the disease is not evaluated.

US 2018/203020 A1 (CHU DE NICE et al.) 19 July 2018 (2018-07-19) discloses autoantibodies to PLA2R1 as marker for prognosis of PN but it does not disclose EXT1/2 as marker for PN.

### SUMMARY

The present invention is defined by the independent claims. The dependent claims depict additional embodiments of the invention.

This document provides methods and materials involved in identifying and/or treating mammals (e.g., humans) having membranous nephropathy (e.g., lupus membranous nephritis) likely or unlikely to progress to end stage kidney disease. For example, this document provides methods and materials for identifying a mammal (e.g., a human) having membranous nephropathy (e.g., lupus membranous nephritis) as being likely or unlikely to progress to end stage kidney disease. As described herein, mammals (e.g., humans) having membranous nephropathy (e.g., lupus membranous nephritis) that contain kidney tissue (e.g., GBM) that does not express EXT1 or EXT2 polypeptides (e.g., kidney tissue that is EXT1-negative and EXT2-negative) can be identified as being likely to progress to end stage kidney disease. In such cases, the mammal can be classified as being likely to progress to end stage kidney disease. As also described herein, mammals (e.g., humans) having membranous nephropathy (e.g., lupus membranous nephritis) that contain kidney tissue (e.g., GBM) that expresses EXT1 or EXT2 polypeptides (e.g., kidney tissue that is EXT1-positive and EXT2-positive) can be identified as being unlikely to progress to end stage kidney disease. In such cases, the mammal can be classified as being unlikely to progress to end stage kidney disease. Identifying mammals (e.g., humans) having membranous nephropathy as being likely or unlikely to progress to end stage kidney disease can allow clinicians and patients to proceed with appropriate membranous nephropathy treatment options.

This document also provides methods and materials for treating membranous nephropathy (e.g., lupus membranous nephritis). For example, a mammal (e.g., a human) having membranous nephropathy (e.g., lupus membranous nephritis) that was identified as being likely to progress to end stage kidney disease as described herein can be administered one or more immunosuppressive agents (e.g., corticosteroids, cyclosporine, mycophenolate mofetil or a B-cell reduction or depletion agent such as Rituximab) to reduce the progression of the mammal to end stage kidney disease. Having the ability to administer one or more immunosuppressive agents to mammals (e.g., humans) having membranous nephropathy that was identified as being likely to progress to end stage kidney disease can allow clinicians and patients to treat membranous nephropathy effectively.

In general, one aspect of this document features an *ex vivo* or *in vitro* method for identifying a mammal as having membranous nephropathy that is likely or unlikely to progress to end stage kidney disease. The method comprises (or consists essentially of or consists of) (a) determining if kidney tissue from the mammal expresses a polypeptide, wherein the polypeptide is an exostosin 1 (EXT1) polypeptide or an exostosin 2 (EXT2) polypeptide; (b) classifying the mammal as having the membranous nephropathy that is unlikely to progress to the end stage kidney disease if the kidney tissue expresses the polypeptide; and (c) classifying the mammal as having the membranous nephropathy that is likely to progress to the end stage kidney disease if the kidney tissue does not express the polypeptide. The mammal can be a human. The polypeptide can be the EXT1 polypeptide. The polypeptide can be the EXT2 polypeptide. The method can comprise classifying the mammal as having the membranous nephropathy that is unlikely to progress to the end stage kidney disease. The method can comprise classifying the mammal as having the membranous nephropathy that is likely to progress to the end stage kidney disease. The method can comprise using immunohistochemistry to determine if the kidney tissue expresses the polypeptide. The method can comprise using laser microdissection and mass spectrometry to determine if the kidney tissue expresses the polypeptide. The method can comprise determining that the kidney tissue expresses the EXT1 polypeptide and the EXT2 polypeptide. The method can comprise determining that the kidney tissue does not express the EXT1 polypeptide and the EXT2 polypeptide.

In another aspect, this document features an immunosuppressant for use in a method for treating a mammal having membranous nephropathy likely to progress to end stage kidney disease. The method comprises (or consists essentially of or consists of) (a) determining that kidney tissue from the mammal does not express a polypeptide, wherein the polypeptide is an exostosin 1 (EXT1) polypeptide or an exostosin 2 (EXT2) polypeptide; and (b) administering an immunosuppressant to the mammal to reduce the rate of progression to the end stage kidney disease. The mammal can be a human. The polypeptide can be the EXT1 polypeptide. The polypeptide can be the EXT2 polypeptide. The method can comprise using immunohistochemistry to determine that the kidney tissue does not express the polypeptide. The method can comprise using laser microdissection and mass spectrometry to determine that the kidney tissue does not express the polypeptide. The method can comprise determining that the kidney tissue does not express the EXT1 polypeptide and the EXT2 polypeptide. The immunosuppressant can be a corticosteroid, cyclosporine, mycophenolate mofetil, or a B-cell reduction or depletion agent.

In another aspect, this document features an immunosuppressant for use in a method for treating a mammal having membranous nephropathy likely to progress to end stage kidney disease. The method comprises (or consists essentially of or consists of) administering an immunosuppressant to a mammal identified as having kidney tissue that does not express a polypeptide, wherein the polypeptide is an exostosin 1 (EXT1) polypeptide or an exostosin 2 (EXT2) polypeptide. The mammal can be a human. The polypeptide can be the EXT1 polypeptide. The polypeptide can be the EXT2 polypeptide. The method can comprise using immunohistochemistry to determine that the kidney tissue does not express the polypeptide. The method can comprise using laser microdissection and mass spectrometry to determine that the kidney tissue does not express the polypeptide. The method can comprise determining that the kidney tissue does not express the EXT1 polypeptide and the EXT2 polypeptide. The immunosuppressant can be a corticosteroid, cyclosporine, mycophenolate mofetil, or a B-cell reduction or depletion agent.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict between the present specification and a reference mentioned herein, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

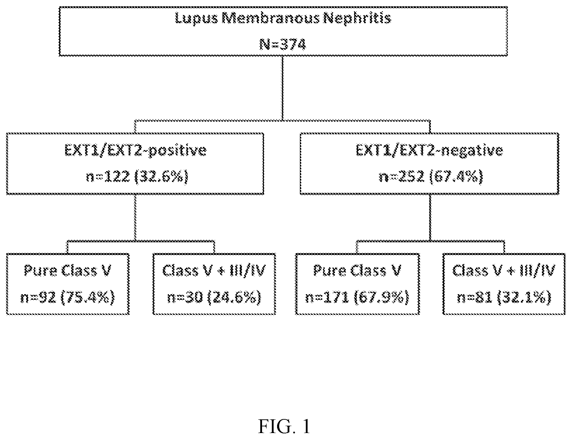

Figure 1. Patient cohort of lupus membranous nephritis (LMN) with and without class III/IV lupus nephritis.

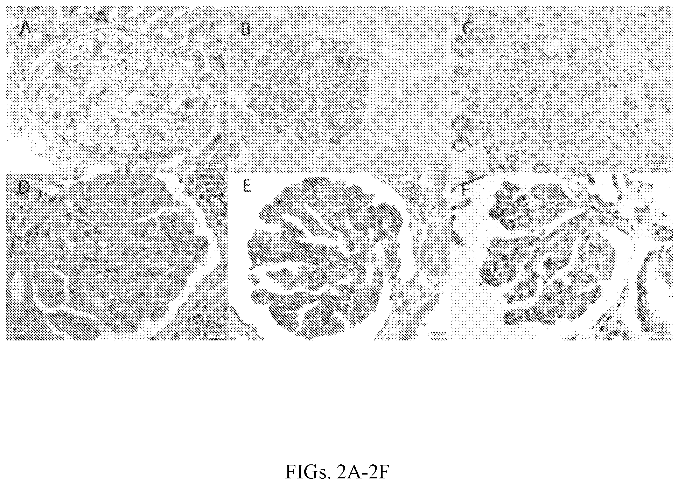

Figures 2A-F. Light Microscopy and immunohistochemistry (IHC) of EXT1/EXT2-positive lupus membranous nephritis (LMN). Top panels (A-C) show pure class V, and bottom panel (D-F) show class V and class III proliferative LN. Figures 2B and 2E show IHC for EXT1, and Figures 2C and 2F show IHC for EXT2 (40x).

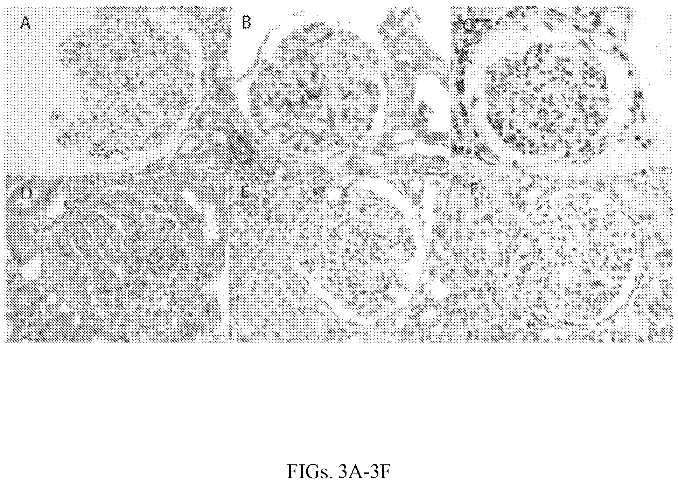

Figures 3A-F. Light Microscopy and immunohistochemistry (IHC) of EXT1/EXT2-negative lupus membranous nephritis (LMN). Light microscopy - Top panels (A-C) show pure class V, and bottom panels (D-F) show class V and class III proliferative LN. Figures 3B and 3E show IHC for EXT1, and Figures 3C and 3F show IHC for EXT2 (40x).

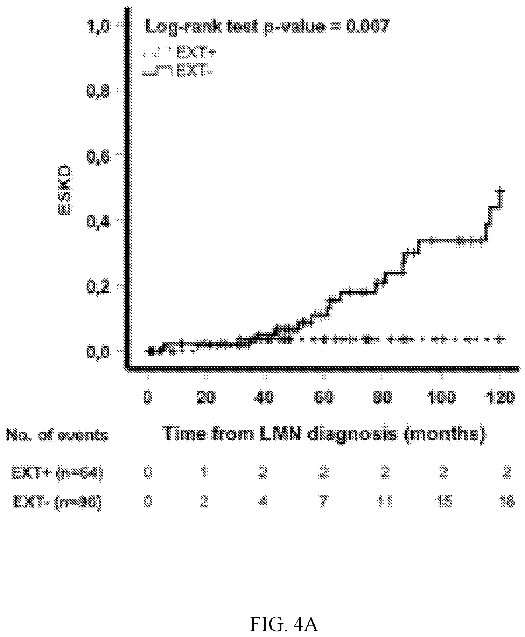

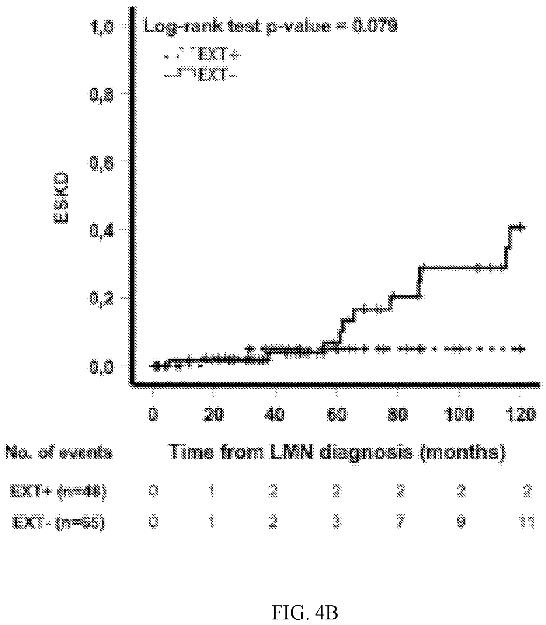

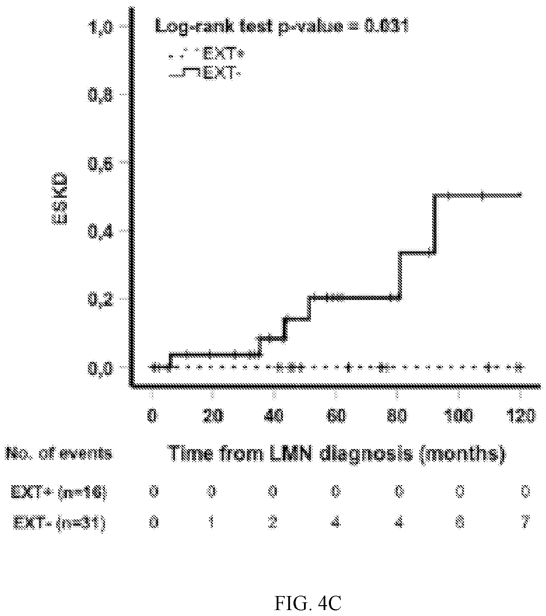

Figures 4A-C. Cumulative incidence of ESKD in patients with lupus membranous nephritis (LMN). Kaplan-Meier plots of the cumulative incidence of ESKD over 10 years. Figure 4A: EXT1/EXT2-positive and EXT1/EXT2-negative LMN (± class III/IV LN): 64 EXT+ versus 96 EXT-, 2 versus 18 events, P=0.007. Figure 4B: EXT1/EXT2-positive and EXTl/EXT2-negative pure class V LMN (with no class III/IV LN): 48 EXT+ versus 65 EXT-, 2 versus 11 events, P=0.079. Figure 4C: EXT1/EXT2-positive and EXT1/EXT2-negative class V LMN + class III/IV LN: 16 EXT+ versus 31 EXT-, 0 versus 7 events, P=0.031. Abbreviations: ESKD = end stage kidney disease; EXT = exostosin.

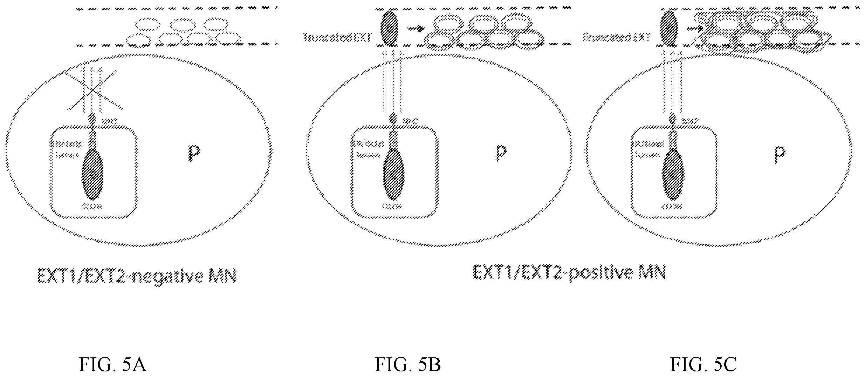

Figures 5A-C. Schematic of EXT1/EXT2-positive and EXT1/EXT2-negative lupus membranous nephritis (LMN). Figure 5A. EXT1/EXT2-negative LMN. There is no increased secretion of EXT1/EXT2 into the GBM. Figures 5B-C. EXT1/EXT2-positive LMN. Figure 5B: In EXT1/EXT2-positive LMN, there is increased secretion of the catalytic domain of exostosin into the GBM that is part of the immune complexes. In Figure 5C: Next, EXT1/EXT2 generate more heparan sulfates with the GBM and those coating the immune complexes. Abbreviations: P = podocyte; C = catalytic domain of exostosin; S = stalk; red circle = exostosin; blue lines = increased expression of heparan sulfated in the GBM and around the deposits; green = immune complexes; dash lines = glomerular basement membranes.

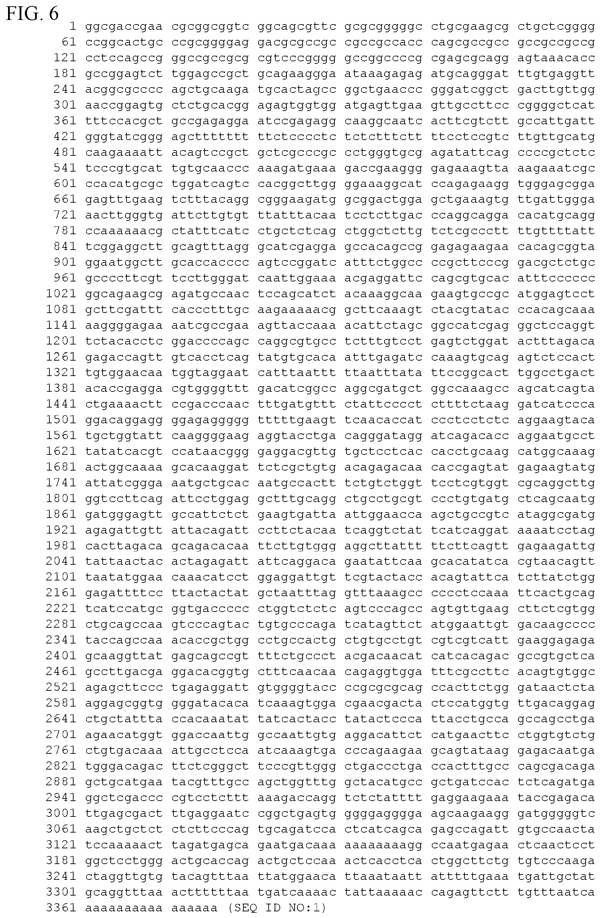

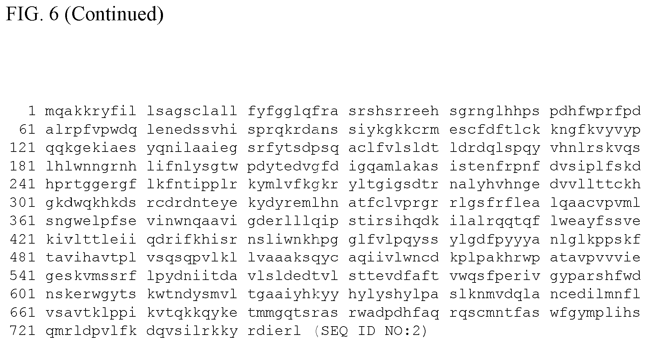

Figure 6 is a sequence listing of a nucleic acid sequence (SEQ ID NO: 1) encoding a human EXT1 polypeptide and an amino acid sequence (SEQ ID NO:2) of a human EXT1 polypeptide.

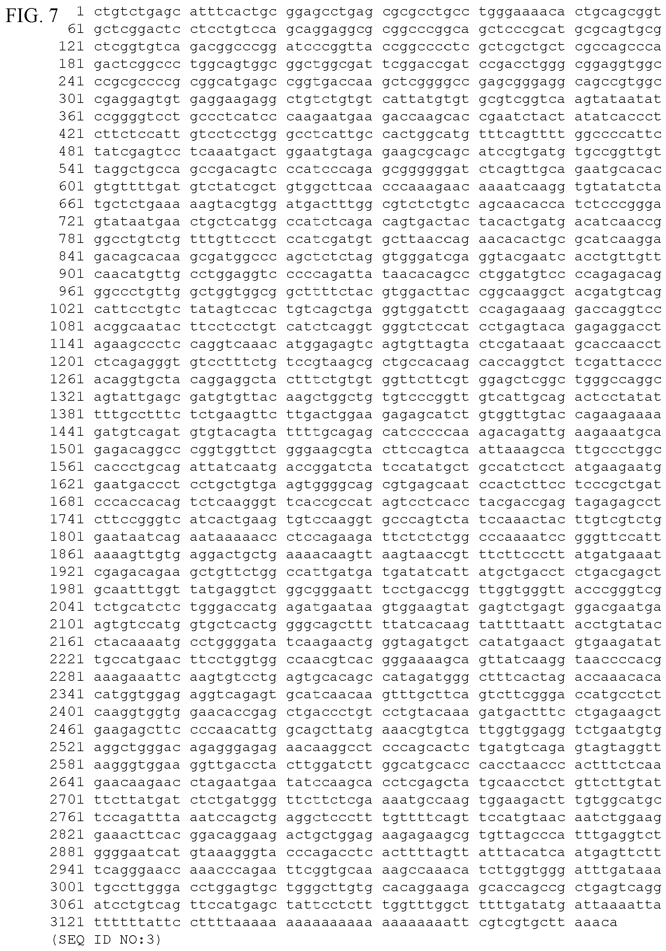

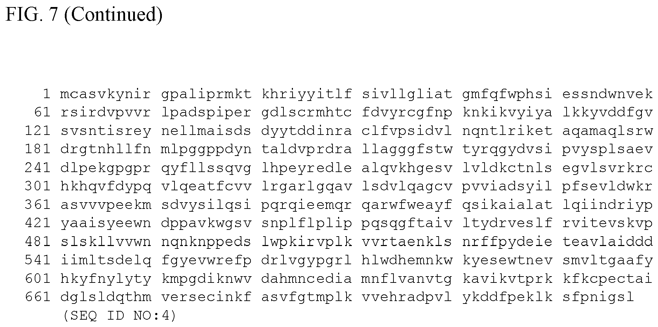

Figure 7 is a sequence listing of a nucleic acid sequence (SEQ ID NO:3) encoding a human EXT2 polypeptide and an amino acid sequence (SEQ ID NO:4) of a human EXT2 polypeptide.

### DETAILED DESCRIPTION

This document provides methods and materials for identifying and/or treating mammals (e.g., humans) having membranous nephropathy (e.g., lupus membranous nephritis). For example, this document provides methods and materials for identifying a mammal (e.g., a human) having membranous nephropathy (e.g., lupus membranous nephritis) as being likely or unlikely to progress to end stage kidney disease.

Any appropriate mammal having membranous nephropathy can be identified as being likely or unlikely to progress to end stage kidney disease. For example, humans and other primates such as monkeys having membranous nephropathy can be identified as being likely or unlikely to progress to end stage kidney disease. In some cases, dogs, cats, horses, cows, pigs, sheep, mice, or rats having membranous nephropathy can be identified as being likely or unlikely to progress to end stage kidney disease as described herein.

As described herein, mammals (e.g., humans) having membranous nephropathy that have kidney tissue (e.g., GBM) that does not express EXT1 or EXT2 polypeptides can be identified as being likely to progress to end stage kidney disease, and mammals (e.g., humans) having membranous nephropathy (e.g., lupus membranous nephritis) that contain kidney tissue (e.g., GBM) that expresses EXT1 or EXT2 polypeptides (e.g., kidney tissue that is EXT1-positive and EXT2-positive) can be identified as being unlikely to progress to end stage kidney disease.

Any appropriate method can be used to determine if a mammal (e.g., a human) having membranous nephropathy expresses or does not express EXT1 or EXT2 polypeptides. For example, immunological techniques such as immunohistochemistry (IHC) techniques, immunofluorescence (IF) techniques, or Western blot techniques can be used to determine if a mammal (e.g., a human) has kidney tissue (e.g., GBM) that expresses or does not express EXT1 or EXT2 polypeptides. In some cases, a kidney tissue sample obtained from a mammal to be tested can be stained using an anti-EXT1 antibody to determine if the mammal has kidney tissue (e.g., GBM) that expresses or does not express EXT1 polypeptides. In some cases, a kidney tissue sample obtained from a mammal to be tested can be stained using an anti-EXT2 antibody to determine if the mammal has kidney tissue (e.g., GBM) that expresses or does not express EXT2 polypeptides. Any appropriate sample can be used to determine if a mammal (e.g., a human) has kidney tissue (e.g., GBM) that expresses or does not express EXT1 or EXT2 polypeptides. For example, kidney tissue biopsies can be obtained from a mammal (e.g., a human) being tested and used to determine if a mammal (e.g., a human) has kidney tissue (e.g., GBM) that expresses or does not express EXT1 or EXT2 polypeptides.

As used herein, kidney tissue is considered to express a polypeptide (e.g., an EXT1 polypeptide or an EXT2 polypeptide) when the GBM shows granular staining for the polypeptide (e.g., an EXT1 polypeptide or an EXT2 polypeptide) on immunohistochemistry or immunofluorescence microscopy using an anti-EXT1 antibody or an anti-EXT2 antibody. As used herein, kidney tissue is considered to not express a polypeptide (e.g., an EXT1 polypeptide or an EXT2 polypeptide) when the GBM shows no granular staining for the polypeptide (e.g., an EXT1 polypeptide or an EXT2 polypeptide) on immunohistochemistry or immunofluorescence microscopy using an anti-EXT1 antibody or an anti-EXT2 antibody.

A human EXT1 polypeptide can have the amino acid sequence set forth in Figure 1. A human EXT2 polypeptide can have the amino acid sequence set forth in Figure 2.

Once a mammal (e.g., a human) having membranous nephropathy is identified as having kidney tissue that does not express an EXT1 polypeptide and/or an EXT2 polypeptide (e.g., EXT1-negative and EXT2-negative kidney tissue) as described herein, the mammal can be classified as having membranous nephropathy likely to progress to end stage kidney disease. Once a mammal (e.g., a human) having membranous nephropathy is identified as having kidney tissue that expresses an EXT1 polypeptide and/or an EXT2 polypeptide (e.g., EXT1-positive and EXT2-positive kidney tissue) as described herein, the mammal can be classified as having membranous nephropathy unlikely to progress to end stage kidney disease.

As described herein, this document also provides methods and materials for treating a mammal having membranous nephropathy likely to progress to end stage kidney disease. For example, a mammal (e.g., a human) having membranous nephropathy that was identified as likely to progress to end stage kidney disease as described herein can be treated with one or more immunosuppressants. **In** some cases, a mammal (e.g., a human) having membranous nephropathy that was identified as being likely to progress to end stage kidney disease as described herein can be administered, or instructed to self-administer, one or more immunosuppressants (e.g., anti-CD20 antibodies such as rituximab) to treat membranous nephropathy and/or slow the progression to end stage kidney disease.

Any appropriate immunosuppressant can be administered to a mammal (e.g., a mammal that was identified as being likely to progress to end stage kidney disease and/or that was identified as having EXT1-negative and/or EXT2-negative kidney tissue) to treat membranous nephropathy and/or slow the progression to end stage kidney disease. In some cases, an immunosuppressant used as described herein to treat membranous nephropathy and/or slow the progression to end stage kidney disease can reduce inflammation and/or reduce B-cell autoantibody production within a mammal. Examples of immunosuppressants that can be used as described herein to treat membranous nephropathy and/or slow the progression to end stage kidney disease include, without limitation, mycophenolate mofetil (e.g., Cellcept); steroids such as prednisone; B-cell inhibitors such as anti-CD20 antibodies (e.g., rituximab); calcineurin inhibitors such as cyclosporine and tacrolimus; and alkylating agents/chemotherapeutic drugs such as cyclophosphamide.

In some cases, two or more (e.g., two, three, four, five, six, or more) immunosuppressants can be administered to a mammal having membranous nephropathy that was identified as being likely to progress to end stage kidney disease as described herein. For example, two immunosuppressants (e.g., prednisone and Cellcept) can be administered to a human having membranous nephropathy that was identified as being likely to progress to end stage kidney disease as described herein.

In some cases, one or more immunosuppressants can be administered to a mammal once or multiple times over a period of time ranging from days to months. In some cases, one or more immunosuppressive drugs can be given to achieve remission of membranous nephropathy, and then given during follow up periods to prevent relapse of the membranous nephropathy. In some cases, one or more immunosuppressants can be formulated into a pharmaceutically acceptable composition for administration to a mammal (e.g., a human) having membranous nephropathy that was identified as being likely to progress to end stage kidney disease as described herein to reduce inflammation and/or to reduce B-cell autoantibody production within that mammal. For example, a therapeutically effective amount of an immunosuppressant can be formulated together with one or more pharmaceutically acceptable carriers (additives) and/or diluents. A pharmaceutical composition can be formulated for administration in solid or liquid form including, without limitation, in the form of sterile solutions, suspensions, sustained-release formulations, tablets, capsules, pills, powders, or granules.

Pharmaceutically acceptable carriers, fillers, and vehicles that can be used in a pharmaceutical composition described herein can include, without limitation, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

A pharmaceutical composition containing one or more immunosuppressants can be designed for oral or parenteral (including subcutaneous, intramuscular, intravenous, and intradermal) administration. When being administered orally, a pharmaceutical composition can be in the form of a pill, tablet, or capsule. Compositions suitable for parenteral administration can include aqueous and nonaqueous sterile injection solutions that can contain anti-oxidants, buffers, bacteriostats, and solutes that render the formulation isotonic with the blood of the intended recipient. The formulations can be presented in unit-dose or multi-dose containers, for example, sealed ampules and vials, and can be stored in a freeze dried (lyophilized) condition requiring the addition of the sterile liquid carrier, for example, water for injections, immediately prior to use. Extemporaneous injection solutions and suspensions can be prepared from sterile powders, granules, and tablets.

In some cases, a pharmaceutically acceptable composition including one or more immunosuppressants can be administered locally or systemically. For example, a composition provided herein can be administered locally by intravenous injection or blood infusion. In some cases, a composition provided herein can be administered systemically, orally, or by injection to a mammal (e.g., a human).

Effective doses can vary depending on the severity of the nephropathy, the route of administration, the age and general health condition of the subject, excipient usage, the possibility of co-usage with other therapeutic treatments, and the judgment of the treating physician.

An effective amount of a composition containing one or more immunosuppressants can be any amount that slows the progression to end stage kidney disease and/or reduces inflammation or B-cell autoantibody production (e.g., B-cell antibody production inhibition or reduction in the number of B-cells) within a mammal having membranous nephropathy without producing significant toxicity to the mammal. For example, an effective amount of rituximab to treat membranous nephropathy that was identified as being likely to progress to end stage kidney disease as described herein can be from about 500 mg to about 1.5 g (e.g., from about 500 mg to about 1.25 g, from about 500 mg to about 1.0 g, from about 500 mg to about 750 mg, from about 750 mg to about 1.5 g, from about 1 g to about 1.5 g, or from about 1.25 g to about 1.5 g) administered intravenously about two weeks apart. In some cases, an effective amount of rituximab to treat membranous nephropathy that was identified as being likely to progress to end stage kidney disease as described herein can be from about 200 mg/m² to about 500 mg/m² (e.g., from about 200 mg/m² to about 450 mg/m², from about 200 mg/m² to about 400 mg/m², from about 200 mg/m² to about 375 mg/m², from about 250 mg/m² to about 500 mg/m², from about 300 mg/m² to about 500 mg/m², from about 350 mg/m² to about 500 mg/m², or from about 350 mg/m² to about 400 mg/m²) administered weekly for about four weeks. If a particular mammal fails to respond to a particular amount, then the amount of an immunosuppressant can be increased by, for example, two fold. After receiving this higher amount, the mammal can be monitored for both responsiveness to the treatment and toxicity symptoms, and adjustments made accordingly. In some cases, the effective amount of a composition containing one or more immunosuppressants can remain constant or can be adjusted as a sliding scale or variable dose depending on the mammal's response to treatment. Various factors can influence the actual effective amount used for a particular application. For example, the frequency of administration, duration of treatment, use of multiple treatment agents, route of administration, and severity of the condition can require an increase or decrease in the actual effective amount administered.

The frequency of administration of one or more immunosuppressants can be any amount that slows the progression to end stage kidney disease and/or reduces inflammation or B-cell autoantibody production (e.g., B-cell antibody production inhibition or reduction in the number of B-cells) within a mammal having membranous nephropathy without producing significant toxicity to the mammal. For example, the frequency of administration of an immunosuppressant can be from about once a day to about once a month (e.g., from about once a week to about once every other week). The frequency of administration of one or more immunosuppressants can remain constant or can be variable during the duration of treatment. A course of treatment with a composition containing one or more immunosuppressants can include rest periods. For example, a composition containing one or more immunosuppressants can be administered daily over a two-week period followed by a two-week rest period, and such a regimen can be repeated multiple times. As with the effective amount, various factors can influence the actual frequency of administration used for a particular application. For example, the effective amount, duration of treatment, use of multiple treatment agents, route of administration, and severity of the condition may require an increase or decrease in administration frequency.

An effective duration for administering a composition containing one or more immunosuppressants can be any duration that slows the progression to end stage kidney disease and/or reduces inflammation or B-cell autoantibody production (e.g., B-cell antibody production inhibition or reduction in the number of B-cells) within a mammal having membranous nephropathy without producing significant toxicity to the mammal. In some cases, the effective duration can vary from several days to several months. In general, the effective duration for administering a composition containing one or more immunosuppressants to treat membranous nephropathy can range in duration from about one month to about five years (e.g., from about two months to about five years, from about three months to about five years, from about six months to about five years, from about eight months to about five years, from about one year to about five years, from about one month to about four years, from about one month to about three years, from about one month to about two years, from about six months to about four years, from about six months to about three years, or from about six months to about two years). In some cases, the effective duration for administering a composition containing one or more immunosuppressants to treat membranous nephropathy can be for as long as the mammal is alive. Multiple factors can influence the actual effective duration used for a particular treatment. For example, an effective duration can vary with the frequency of administration, effective amount, use of multiple treatment agents, route of administration, and severity of the condition being treated.

In some cases, a course of treatment and/or the severity of one or more symptoms related to membranous nephropathy can be monitored. Any appropriate method can be used to determine whether or not membranous nephropathy is being treated and/or to determine whether or not progression to end stage kidney disease is being slowed. For example, remission and relapse of the disease can be monitored by testing for one or more markers for membranous nephropathy. In some cases, remission can be ascertained by detecting the disappearance or reduction of autoantibodies to THS7DA, NELL1, Sema3B, and/or PLA2R in the sera. In some cases, relapse of membranous nephropathy can be ascertained by a reappearance or elevation of autoantibodies to THS7DA, NELL1, Sema3B, and/or PLA2R in the sera.

The invention will be further described in the following examples, which do not limit the scope of the invention described in the claims.

### EXAMPLES

### Example 1 - Exostosin-positive and Exostosin-negative represent two different phenotypes of Membranous Lupus Nephritis

### Study Design

Patients with a diagnosis of LMN on kidney biopsies were screened. The clinical information was obtained from the electronic medical records for the in-house biopsies. For other biopsies received from outside institutions, clinical information was limited and therefore, medical records with pertinent clinical information were requested.

### Patient characteristics and kidney biopsy data

Patients with clinical history of systemic lupus erythematosus (SLE) and biopsy-proven LMN were further analyzed. Light microscopy, immunofluorescence microscopy, and electron microscopy were performed in each case. Lupus nephritis (LN, Class III and IV) was classified according to the ISN/RPS classification (Bajema et al., Kidney Int., 93:789-796 (2018)). All these patients had paraffin-embedded tissue blocks available. Immunohistochemistry (IHC) studies were performed on 4 micron tissue sections using antibodies against EXT1/EXT2 as described elsewhere (Sethi et al., J. Am. Soc. Nephrol., 30:1123-1136 (2019)). Laser microdissection and mass spectrometry (MS/MS) were performed on a subset of these cases. Details of MS/MS are described elsewhere (Tomas et al., N. Engl. J. Med., 371:2277-2287 (2014)).

### Statistical analysis

Categorical variables were presented as counts (percent) while continuous variables were presented as mean (standard deviation) if they were normally distributed as determined by Shapiro-Wilk test, or as median (interquartile range [IQR]) if non-normal. For comparisons of categorical variables between groups, the Pearson's chi-square test was used if the number of elements in each cell was ≥5; Fisher's exact test was used otherwise. For comparison of continuous variables between groups, an unpaired Student's t-test for independent samples was used for distributions consistent with normality as above, and the Mann-Whitney U test was used otherwise.

The Kaplan Meier method was used to assess cumulative incidence of end-stage kidney disease (ESKD) during the course of the follow-up. ESKD was defined as dependence of renal replacement therapy. Cox proportional hazards regression models were used to determine predictive factors of the ESKD. The hazard ratio (HR) was report with a 95% confidence intervals CI when appropriate.

P-values less than 0.05 (2-sided) were considered significant. IBM^{®} SPSS^{®} Statistics for MacOS, version 25 (IBM, Armonk, NY, USA) was used for data analysis.

### Results

A total of 374 cases with adequate tissue for IHC were included in the study. All patients had a clinical history of SLE. The kidney biopsy specimens of all LMN cases showed the characteristic findings of thickened GBM on light microscopy, bright IgG and C3 staining along the capillary wall on immunofluorescence microscopy, and subepithelial deposits on electron microscopy.

Of 374 biopsies, 122 (32.6%) biopsies had positive EXT1/EXT2 staining on IHC, and 252 (67.4%) were negative for EXT1/EXT2 (Figure 1). IHC showed moderate to intense (2-3+/3) granular staining for both EXT1 and EXT2 along the GBM in the positive cases, and no staining in the negative cases. There were no intermediate cases of trace or minimal staining. EXT1 staining tends to be a little brighter when compared to EXT2 in the positive cases.

### EXT1/EXT2-positive LMN

Of the 122 patients, the median age at the time of kidney biopsy was 32 years (IQR: 25-41) (Table 1A). The majority were female (n=106; 86.9%) patients. At presentation, the median serum creatinine (SCr) and proteinuria were 0.8 mg/dL (IQR: 0.6-1.2; n=112) and 3.5 g/24 hours (IQR: 2-6.6; n=95), respectively. Fifty-seven (60%, n=95) patients presented with proteinuria ≥3.5 g/day at the time of kidney biopsy. Forty-one (33.6%) patients had hematuria.

**Table 1A. Clinicopathologic features of EXT1/EXT2-positive and EXT1/EXT2-negative lupus membranous nephritis.**

| **Variable** | **EXT1/EXT2-positive, n=122** | **EXT1/EXT2-negative, n=252** | ****P*-value** |
|---|---|---|---|
| **Age, years** | Median: 32 | Median: 35 | **0.013** |
| | IQR: 25-41 | IQR: 27-45 | |
| **Sex** | Male: 16 (13.1%) | Male: 47 (18.7%) | 0.180 |
| | Female: 106 (86.9%) | Female: 205 (81.3%) | |
| **Serum Creatinine at presentation, mg/dL** | Median: 0.8 | Median: 0.9 | **0.022** |
| | IQR: 0.6-1.2; n=112 | IQR: 0.7-1.4; n=227 | |
| **Proteinuria at presentation, g/24 hours** | Median: 3.5 | Median: 3 | 0.081 |
| | IQR: 2-6.6; n=95 | IQR: 1.8-4.8; n=199 | |
| **Proteinuria ≥3.5 g/day at presentation** | 57 (60%) n=95 | 87 (43.7%), n=199 | **0.009** |
| **Hematuria** | 41 (33.6%) | 87 (34.5%) | 0.861 |
| **Other autoimmune disease** | 32 (26.2%) | 47 (18.7%) | 0.092 |
| **Sclerosed glomeruli** (%) | Median: 3.8 | Median: 9.1 | **0.001** |
| | IQR: 0-12.1 | IQR: 0-24.8 | |
| **Interstitial fibrosis and tubular atrophy (%)** | Median: 0 | Median: 10 | **<0.0001** |
| | IQR: 0-10 | IQR: 5-20 | |
| **Proliferative features on light microscopy** | 30 (24. 6%) | 81 (32.1%) | 0.134 |
| **Immunofluorescence (Glomerular staining)** | **Total cases with IF (n=118)** | **Total cases with IF (n=244)** | - |
| | **IgG:** 2-3+ (n=118, 100%) **IgA** (n=89, 75.4%); trace (n=17); 1+ (n=22); 2-3+ (50) | **IgG** (n=244, 100%): trace (n=3); 1+ (n=8); 2-3+ (233) | |
| | | **IgA** (n=186, 76.2%): trace (n=35); 1+ (n=53); 2-3+ (98) | |
| | **IgM** (n=103, 87.3%): trace (n=23); 1 + (n=32); 2-3+ (48) | | |
| | | **IgM** (n=220, 90.2%): trace (n=27); 1+(n=75); 2-3+ (118) | |
| | **C1q** (n=107, 90.7%): trace (n=18), 1+ (n=20); 2-3+ (69) | | |
| | | **C1q** (n=216, 88.5%): trace (n=38), 1+(n=39); 2-3+ (139) | |
| | **C3** (n=117, 99.2%): trace (n=2); 1 + (n=6); 2-3+ (109) | | |
| | | **C3** (n=238, 97.5%): trace (n=11); 1 + (n=29); 2-3+ (198) | |
| | **Kappa light chain** (n=118, 100%): trace (n=3); 1 + (n=8); 2-3+ (107) | | |
| | | **Kappa light chain** (n=241, 98.8): trace (n=12); 1+ (n=31); 2-3+ (198) | |
| | **Lambda light chain** (n=118, 100%): trace (n=1); 1+ (n=1); 2-3+ (116) | | |
| | | **Lambda light chain**(n=241, 98.8%): trace (n=10); 1 + (n=22); 2-3+ (209) | |
| **Electron Microscopy** | **Subepithelial deposits:** 122 (100%), of which 62 (50.8%) also had intramembranous deposits | **Subepithelial deposits:** 250 (99.2%), of which 153 (61.2%) also had intramembranous deposits; Intramembranous deposits only: 2 (0.8%) | - |
| | **Mesangial/Paramesangi al deposits:** 116 (95.1%) | | |
| | | **Mesangial/Paramesang ial deposits:** 239 (94.8%) | |
| | **Subendothelial deposits:** 71 (58.2%) | | |
| | **Tubuloreticular inclusions:** 107 (87.7%) | **Subendothelial deposits:** 146 (57.9%) | |
| | | **Tubuloreticular inclusions:** 199 (79.0%) | |

| | | | |
|---|---|---|---|
| IQR: interquartile range. *P-value < 0.05 is considered significant | | | |

Thirty-two (26.2%) patients had other associated autoimmune disorders including Grave's disease, antiphospholipid antibody syndrome, mixed connective tissue disease, rheumatoid arthritis, Sjögren syndrome, CREST (*calcinosis,* Raynaud phenomenon, esophageal dysmotility, sclerodactyly, telangiectasia) and/or tested positive for other autoimmune serologies including anti-Smith, anti-SSA, anti-SSB, anti-ribonucleoprotein, anti-nuclear cytoplasmic antibodies, anti-Scl 70, anti-histone and/or anti-chromatin antibodies, lupus anticoagulant, respectively. Complement data were available in 62 patients, of which thirty-one (50%) had low C3 and C4, 4 (6.5%) low C3 only, 3 (4.8%) low C4 only, and the remaining 24 (38.7%) had normal complement levels.

Ninety-two (75.4%) biopsies showed pure class V LMN and remaining 30 (24.6%) biopsies had mixed class V with proliferative features (19 Class III and 11 Class IV LN, Table 1A). The kidney biopsy specimens of all cases of EXT1/EXT2-positive LMN showed the characteristic findings of thickened GBM on light microscopy (Figure 2). Overall, on average 16.6 glomeruli (SD ± 10.0) were present with a median of 3.8% global glomerulosclerosis (IQR: 0-12.1). Interstitial fibrosis and tubular atrophy (IFTA) was minimal (<10%) in 89 (73%), mild (10-25%) in 21 (17.2%), moderate (26-50%) in 8 (6.6%), and severe (>50%) in 4 (3.3%) cases, respectively. Overall the median IFTA was 0% (IQR: 0-10). Immunofluorescence studies were performed in 118 of 122 biopsies where sufficient glomeruli were present for evaluation. Of these 118 biopsies, all demonstrated bright IgG (2-3+/3), 89 showed IgA (trace-3+/3), 103 showed IgM (trace-3+/3), 118 showed kappa and lambda light chains (trace-3+/3), 107 showed C1q (trace-3+/3), and 117 cases showed C3 (trace-3+/3). Immunofluorescence study for PLA2R was evaluated in 10 cases, and all were negative. On electron microscopy, 8 (6.6%) cases were class I, 58 (47.5%) cases were class II, 56 cases (45.9%) were class III, and none of the cases was class IV based on Ehrenreich and Churg classification (Table 1B). Subendothelial deposits and mesangial/paramesangial deposits were present in 71 (58.2%) and 116 (95.1%) of 122 cases, respectively. Tubuloreticular inclusions were present in the endothelial cells in 107 (87.7%) cases. Laser microdissection and mass spectrometry was performed in a subset of cases (n=8), which showed high spectral counts for EXT1 (average: 88.6, SD: ± 37.2) and EXT2 (average: 66.1, SD: ± 34.6), thus confirming the IHC findings (data not shown).

**Table 1B: Electron microscopy class based on Ehrenreich and Churg Classification.**

| **Ehrenreich and Churg Classification** | EXT1/EXT2 negative n = 252 (67.4) | EXT1/EXT2 positive n = 122 (32.6) | *P-value |
|---|---|---|---|
| I | 9 (3.6%) | 8 (6.6%) | 0.194 |
| II | 72 (28.6%) | 58 (47.5%) | <0.0001 |
| III | 167 (66.3%) | 56 (45.9%) | <0.0001 |
| IV | 3 (1.2%) | 0 (0.0%) | 0.226 |

### EXT1/EXT2-negative LMN

Of the 252 patients, the median age at the time of kidney biopsy was 35 years (IQR: 27-45) (Table 1A). The majority were female (n=205; 81.3%). At the time of kidney biopsy, the median SCr and urine proteinuria were 0.9 mg/dL (IQR: 0.7-1.4; n=227) and 3 g/24 hours (IQR: 1.8-4.8; n=199), respectively. Eighty-seven (43.7%, n=199) patients presented with proteinuria ≥3.5 g/day at the time of kidney biopsy. Eighty-seven (34.5%) patients had hematuria.

Forty-seven (18.7%) patients had other associated autoimmune disorders (rheumatoid arthritis, Sjögren syndrome, Graves' disease, mixed connective tissue disease, immune thrombocytopenic purpura) and/or tested positive for other autoimmune serologies including anti-Smith, rheumatoid factor, anti-SSA, anti-SSB, anti-ribonucleoprotein, anti-nuclear cytoplasmic antibodies, anti-Scl 70, and anti-histone antibodies, respectively. Complement data were available in 126 patients, of which 62 (49.2%) had low C3 and C4, 20 (15.9%) low C3 only, 11 (8.7%) low C4 only, and the remaining 33 (26.2%) had normal complement levels.

A total of 171 (67.9%) cases showed pure class V MN, and the remaining 81 (32.1%) cases had mixed class V with proliferative features (29 Class III and 52 Class IV lupus nephritis). The kidney biopsy specimens of all cases of EXT1/EXT2-negative LMN showed the characteristic findings of thickened GBM on light microscopy (Figure 3). Overall, on average of 19.3 glomeruli (SD ± 11) were present with a median of 9.1% global glomerulosclerosis (IQR: 0-24.8). Interstitial fibrosis and tubular atrophy was minimal (<10%) in 120 (47.6%), mild (10-25%) in 91 (36.1%), moderate (26-50%) in 34 (13.5%), and severe (>50%) in 7 (2.8%) cases, respectively. Overall, the median IFTA was 10% (IQR: 5-20). Immunofluorescence studies were performed in 244 of 252 biopsies where sufficient glomeruli were present for evaluation. Of these 244 biopsies, all demonstrated IgG, 238 cases showed C3 (trace-3+/3), 216 showed C1q (trace-3+/3), 220 showed IgM (trace-3+/3), 186 showed IgA (trace-3+/3), and 241 showed kappa and lambda light chains (trace-3+/3), respectively. Immunofluorescence study for PLA2R was evaluated in 10 cases, and all were negative. On electron microscopy, 9 (3.6%) cases were class I, 72 (28.6%) cases were class II, 167 cases (66.3%) were class III, and 3 (1.2%) cases were class IV based on Ehrenreich and Churg classification (Table 1B).

Subendothelial and mesangial/paramesangial deposits were present in 146 (57.9%) and in 239 (94.8%) of 252 cases, respectively. Tubuloreticular inclusions were present in 199 (79%). Laser microdissection and mass spectrometry was performed in a subset of cases (n=7) and was negative for EXT 1 and EXT2, thus confirming the IHC findings (data not shown).

The clinical and laboratory features comparing EXT1/EXT2-positive and EXT1/EXT-2 negative LMN patients are described in Table 1A. In summary, compared to EXT1/EXT2-negative cohort (n=252), EXT1/EXT2-positive LMN (n=122) patients were slightly younger (*P*=0.013), have lower serum creatinine levels (*P*=0.022)*,* tend to present with proteinuria ≥3.5 gms/24 hours (*P*=0.009), and have less chronicity (glomerulosclerosis: *P*=0.001; interstitial fibrosis and tubular atrophy: *P*<0.0001). Based on Ehrenreich and Churg classification, a higher proportion of EXT1/EXT2-negative LMN have class III lesions compared to EXT1/EXT2-positive LMN (66.3% vs 45.9%, *P*<0.0001). On the other hand, a higher proportion of EXT1/EXT2-positive LMN have class II lesions compared to EXT1/EXT2-negative LMN (47.5% vs 28.6%, *P*<0.0001).

### EXT1/EXT2 in proliferative lupus nephritis (LN) without LMN

38 cases of class I-IV (class I: n=1; class II: n=7; class III: n=6; class IV; n=24) lupus nephritis with no component of LMN also were stained. All cases were negative for EXT1/EXT2 on IHC.

### Re-biopsy of LMN

During the course of follow-up, four LMN cases had re-biopsy after the initial diagnosis of LMN; three of which were EXT1/EXT2-positive and one was EXT1/EXT2-negative LMN. All three biopsies of EXT-positive LMN did not show significant progression of chronicity indexes, while the single EXT1/EXT2-negative LMN showed increased global glomerulosclerosis and IFTA (Table 1C). EXT1 staining done in re-biopsies of four cases showed bright (3+) EXT1 staining in two cases (case 1 and 2) and weak (1+) staining in one case (case 3) of EXT1/EXT2-positive LMN, while it was negative in one EXT1/EXT2-negative LMN. In two EXT1/EXT2-positive LMN cases (case 1 and 2), re-biopsies were done after follow-up of 2 years. In one EXT1/EXT2-positive LMN case, re-biopsy was done after 10 years of follow-up, and in the one EXT1/EXT2-negative LMN case, re-biopsy was done after 7 years of follow-up.

**Table 1C: Chronicity findings in LMN on re-biopsy.**

| | **Glomeruli/ sclerosed** | **IFTA %** | **Follow-up (years)** | **Re-biopsy: Glomeruli/ sclerosed** | **Re-biopsy: IFTA %** |
|---|---|---|---|---|---|
| **Case 1 *** | 17/1 (5.8%) | 0 | 2 | 17/2 (8.5%) | 0 |
| **Case 2 *** | 10/0 (0%) | 0 | 2 | 26/0 (0%) | 0 |
| **Case 3 *** | 3/0 (0%) | 0 | 10 | 42/2 (4.7%) | 0 |
| **Case 4 *** | 7/0(0%) | 0 | 7 | 42/19 (45.2%) | 25 |

| | | | | | |
|---|---|---|---|---|---|
| *Case 1, 2, 3 are EXT1/EXT2-positive LMN, and case 4 is EXT1/EXT2-negative LMN. | | | | | |

### Clinical follow-up of EXT1/EXT2-positive and EXT-negative LMN

Clinical follow-up data were available in a total of 160 patients. Clinical characteristics, follow-up, and outcome data are summarized in Tables 2-4 and Figure 4.

**Table 2. Clinical characteristics, follow-up and outcomes of EXT1/EXT2-positive and EXT1/EXT2-negative lupus membranous nephritis (LMN) with and without LN (combined class V LMN ± class III/IV lupus nephritis)**

| **Variable** | **EXT-positive LMN n = 64/122 (52.5%)** | **EXT- negative LMN n = 96/252 (38.1%)** | ****P*-value** |
|---|---|---|---|
| **Age at presentation, median (IQR) years** | 33 (25-42) | 38 (28-47) | 0.102 |
| **Female, n (%)** | 57 (89.1) | 75 (78.1) | 0.074 |
| **Serum Creatinine at presentation, median (IQR), mg/dL** | 0.80 (0.60-1.00), n=61 | 0.90 (0.70-1.40), n=89 | **0.004** |
| **Proteinuria at presentation, median (IQR), g/24 hours** | 3.9 (1.6-6.7), n=53 | 3.0 (1.5-4.9), n=76 | 0.296 |
| **Proteinuria at presentation ≥3.5 /day, n (%)** | 32 (60.4), n=53 | 33 (43.4), n=76 | 0.058 |
| **Hematuria, n (%)** | 19 (29.7) | 35 (36.5) | 0.375 |
| **Proliferative features, n (%)** | 16 (25.0) | 31 (32.3) | 0.321 |
| **Other autoimmune diseases, n (%)** | 19 (29.7) | 22 (22.9) | 0.337 |
| **Sclerosed glomeruli, median (IQR), %** | 0.0 (0.0 - 10.2) | 13.2 (1.82 - 25.0) | **<0.0001** |
| **Interstitial fibrosis and tubular atrophy, median (IQR), %** | 0.0 (0.0 - 0.0) | 10.0 (5.0 - 20.0) | **<0.0001** |
| **Serum creatinine at end of follow-up, median (IQR), mg/dL** | 0.90 (0.70 - 1.20), n=51 | 1.10 (0.70 - 1.60), n=71 | **0.049** |
| **Proteinuria at end of follow-up, median (IQR), g/24 hours** | 0.85 (0.20 - 2.00), n=36 | 0.90 (0.25 - 2.35), n=57 | 0.887 |
| **Proteinuria at end of follow-up ≥3.5 /day, n (%)** | 3 (8.3), n=36 | 9 (16.1), n=57 | 0.296 |
| **ESKD, n (%)** | 2 (3.1) | 18 (18.8) | 0.003 |
| **Death, n (%)** | 2 (3.1) | 8 (8.3) | 0.182 |
| **Time of follow-up, median (IQR), months** | 48.6 (33.1 - 76.5) | 50.6 (32.7 - 86.8) | 0.848 |

| | | | |
|---|---|---|---|
| **P*-value < 0.05 is considered significant | | | |

**Table 3. Clinical characteristics, follow-up and outcomes of EXT1/EXT2-positive and EXT1/EXT2-negative lupus membranous nephritis (LMN) without proliferative features (Pure Class V)**

| **Variable** | **EXT-positive LMN n = 48/64 (75.0%)** | **EXT-negative LMN n = 65/96 (67.7%)** | ****P*-value** |
|---|---|---|---|
| **Age at presentation, median (IQR) years** | 33 (25 - 42) | 40 (28 - 47) | 0.120 |
| **Female, n (%)** | 42 (87.5) | 53 (81.5) | 0.392 |
| **Serum Creatinine at presentation, median (IQR), mg/dL** | 0.70 (0.60-0.90), n=46 | 0.80 (0.60-1.30),n=58 | 0.010 |
| **Proteinuria at presentation, median (IQR), g/24 hours** | 3.5 (1.5-5.9), n=40 | 2.8 (1.40-4.3), n=51 | 0.230 |
| **Proteinuria at presentation ≥3.5 /day, n (%)** | 23 (57.5), n=40 | 18 (35.3), n=51 | 0.035 |
| **Hematuria, n (%)** | 15 (31.3) | 22 (33.8) | 0.771 |
| **Other autoimmune diseases, n (%)** | 14 (29.2) | 15 (23.1) | 0.464 |
| **Sclerosed glomeruli, median (IQR), %** | 0.0 (0.0 - 8.5) | 9.5 (0.0 - 24.4) | **<0.0001** |
| **Interstitial fibrosis and tubular atrophy, median (IQR), %** | 0.0 (0.0 - 0.0) | 10.0 (5.0 - 25.0) | <0.0001 |
| **Serum Creatinine at end of follow-up, median (IQR), mg/dL** | 0.85 (0.70 - 1.13), n=38 | 1.00 (0.70 -1.57), n=51 | 0.085 |
| **Proteinuria at end of follow-up, median (IQR), g/24 hours** | 0.95 (0.20 - 2.55), n=26 | 0.90 (0.20 - 1.93), n=42 | 0.456 |
| **Proteinuria at end of follow-up ≥3.5 /day, n (%)** | 3 (11.5),n=26 | 6 (14.3), n=42 | 0.745 |
| **ESKD, n (%)** | 2 (4.2) | 11 (16.9) | **0.036** |
| **Death, n (%)** | 2 (4.2) | 2 (3.1) | 0.757 |
| **Time of follow-up, median (IQR), months** | 52.5 (31.6 - 81.2) | 53.6 (34.2 - 97.1) | 0.605 |

| | | | |
|---|---|---|---|
| **P*-value < 0.05 is considered significant | | | |

**Table 4. Clinical characteristics, follow-up and outcomes of all EXT1/EXT2-positive and EXT1/EXT2-negative lupus membranous nephritis (LMN) with proliferative features class V + class III/IV lupus nephritis).**

| **Variable** | **EXT-positive LMN n = 16/64 (25.0%)** | **EXT-negative LMN n = 31/96 (32.3%)** | ****P*-value** |
|---|---|---|---|
| **Age at presentation, median (IQR) years** | 32 (25-43) | 33 (28-47) | 0.387 |
| **Female, n (%)** | 15 (93.8) | 22 (71.0) | 0.071 |
| **Serum Creatinine at presentation, median (IQR), mg/dL** | 1.0 (0.8-2.0), n=15 | 1.20 (0.8-2.3), n=31 | 0.353 |
| **Proteinuria at presentation, median (IQR), g/24 hours** | 6.6 (1.5-7.9), n=13 | 3.7 (1.7-6.5), n=25 | 0.484 |
| **Proteinuria at presentation ≥3.5 /day, n (%)** | 9 (69.2), n=13 | 15 (60), n=25 | 0.576 |
| **Hematuria, n (%)** | 4 (25.0) | 13 (41.9) | 0.252 |
| **Other autoimmune diseases, n (%)** | 5 (31.3) | 7 (22.6) | 0.518 |
| **Sclerosed glomeruli, median (IQR), %** | 4.6 (0.0 - 12.5) | 20.0 (3.22 - 27.8) | **0.013** |
| **Interstitial fibrosis and tubular atrophy, median (IQR), %** | 0.0 (0.0 - 8.75) | 10.0 (5.0 - 20.0) | **0.003** |
| **Serum Creatinine at end of follow-up, median (IQR), mg/dL** | 0.90 (0.8-1.5), n=13 | 1.30 (0.70 - 1.9), n=20 | 0.316 |
| **Proteinuria at end of follow-up, median (IQR), g/24 hours** | 0.60 (0.20 - 1.253), n=10 | 1.5 (0.30 - 3.40), n=15 | 0.115 |
| **Proteinuria at end of follow-up ≥3.5 /day, n (%)** | 0 (0.0), n=10 | 3 (20.0), n=15 | 0.132 |
| **ESKD, n (%)** | 0 (0.0) | 7 (22.6) | 0.039 |
| **Death, n (%)** | 0 (0.0) | 6 (19.4) | 0.060 |
| **Time of follow-up, median (IQR), months** | 48.6 (43.0 - 76.3) | 43.4 (27.1 - 77.9) | 0.266 |

| | | | |
|---|---|---|---|
| *P-value < 0.05 is considered significant | | | |

### Combined class VLMN ± class III/IV LN

Of the 160 patients with follow up, 64 (40%) biopsies were EXT1/EXT2-positive, and 96 (60%) biopsies were EXT1/EXT2-negative LMN (Table 2). The proportion of patients with proliferative features was not statistically significantly different between groups: 16 (25%) of 64 in the EXT1/EXT2-positive group versus 31 (32.3%) of 96 patients in the EXT1/EXT2-negative group showed proliferative features (*P*=0.321). At presentation, the median SCr was statistically significantly higher in EXT1/EXT2-negative patients (0.90 vs. 0.80 mg/dL, *P*=0.004), but there were no differences in the presence of hematuria, or proteinuria ≥3.5 g/day. Kidney biopsies of EXT1/EXT2-negative patients showed a statistically significantly higher median of global glomerulosclerosis and IFTA (*P*<0.0001). The treatment was variable and included the following: i) EXT1/EXT2-postive LMN: five received steroids only, 14 received immunosuppressants, and 22 received a combination of steroids and immunosuppressants, and for the remaining, treatment details were not available; ii) EXT1/EXT2-negative LMN: three were managed conservatively, four received steroids only, 18 received immunosuppressants, and 51 received a combination of steroids and immunosuppressants, and for the remaining, treatment details were not available. The most common immunosuppressants included plaquenil, mycophenolate mofetil, cyclophosphamide, and azathioprine.

Both groups were followed for a similar period of time (48.6 vs 50.6 months, *P*=0.848). At the end of the follow up, patients who were EXT1/EXT2-negative presented with statistically significant higher values of SCr (1.10 vs. 0.90 mg/dL, *P*=0.049). During the course of the disease, patients who were EXT1/EXT2-negative evolved more frequently to ESKD (18.8% vs. 3.1%, *P*=0.003), and the time to this event was statistically significantly shorter in this group when compared to EXT1/EXT2-positive patients (101 vs. 116 months, *P=*0.007, Fig. 4A). There was no difference in death rates between groups. Using multivariable Cox regression, the predictive factors for the development of ESKD at 10 years were male gender (HR 2.92; 95% CI, 1.160-7.344; *P*=0.023) and glomerulosclerosis ≥ 25% (HR 4.028; 95%CI, 1.670-19.714; *P*=0.002) (Table 5).

**Table 5 - Multivariable analysis of predictive factors for ESKD at 10 years.**

| | **Univariable Cox Regression** | | **Multivariable Cox Regression** | |
|---|---|---|---|---|
| **Combined class V LMN ± Class III/IV LN** | **HR (95% CI)** | ***P*-value** | **HR (95% CI)** | ***P*-value** |
| Age | 0.972 (0.938 - 1.014) | 0.212 | | |
| Male | 2.816 (1.119 - 7.082) | **0.028** | 2.918 (1.160 - 7.344) | **0.023** |
| EXT1/EXT2 negative | 5.851 (1.356 - 25.237) | **0.018** | | |
| SCr at presentation | 1.363 (1.103 - 1.685) | **0.004** | | |
| Proteinuria at presentation | 0.961 (0.834 - 1.106) | 0.578 | | |
| Hematuria | 1.145 (0.661 - 1.984) | 0.629 | | |
| Other autoimmune diseases | 1.078 (0.392 - 2.970) | 0.884 | | |
| ≥ 25% glomerulosclerosis | 3.931 (1.632 - 9.468) | **0.002** | 4.028 (1.670-19.714) | **0.002** |
| ≥ 25% IFTA | 4.231 (1.741 - 10.278) | **0.001** | | |

| **Only class V LMN (without class III/IV LN)** | **HR** (95% CI) | ***P*-value** | **HR (95% CI)** | ***P*-value** |
|---|---|---|---|---|
| Age | 0.990 (0.946 - 1.036) | 0.667 | | |
| Male | 2.351 (0.717 - 7.704) | 0.158 | | |
| EXT1/EXT2 negative | 3.556 (0.786 - 16.082) | 0.099 | | |
| SCr at presentation | 1.274 (0.896 - 1.812) | 0.178 | | |
| Proteinuria at presentation | 1.018 (0.905 - 1.145) | 0.767 | | |
| Hematuria | 1.404 (0.660 - 2.986) | 0.378 | | |
| Other autoimmune diseases | 1.285 (0.395 - 4.176) | 0.677 | | |
| ≥ 25% **glomerulosclerosis** | 4.306 (1.440 - 12.876) | **0.009** | | |
| ≥ 25% IFTA | 3.912 (1.305 - 11.728) | **0.015** | | |

| **Class V LMN with class III/IV LN** | **HR (95% CI)** | ***P*-value** | **HR (95% CI)** | ***P*-value** |
|---|---|---|---|---|
| Age | 0.934 (0.841 - 1.037) | 0.203 | | |
| Male | 6.325 (1.031 - 38.799) | **0.046** | 8.350 (1.114 - 62.603) | **0.039** |
| EXT1/EXT2 negative | - | - | - | - |
| SCr at presentation | 1.400 (1.020 - 1.922) | **0.037** | 1.528 (1.039 - 2.246) | **0.031** |
| Proteinuria at presentation | 0.213 (0.015 - 3.037) | 0.254 | | |
| Hematuria | 0.728 (0.303 - 1.745) | 0.476 | | |
| Other autoimmune diseases | 0.851 (0.096 - 7.503) | 0.884 | | |
| ≥ 25% glomerulosclerosis | 2.522 (0.553 - 11.501) | 2.522 | | |
| ≥ 25% IFTA | 6.363 (1.273 - 31.801) | 0.024 | | |

| | | | | |
|---|---|---|---|---|
| Abbreviations: CI = confidence interval; EXT = exostosin; HR = hazard ratio; LMN = lupus membranous nephritis; LN = lupus nephritis; SCr = serum creatinine; IFTA = interstitial fibrosis and tubular atrophy. **P*-value < 0.05 is considered significant. | | | | |

### Only class V LMN (without class III/IV LN)

Forty-eight (75%) of 64 EXT1/EXT2-positive cases and 65 (67.7%) of 96 EXT1/EXT2-negative cases had pure class V with no class III/IV LN (Table 3). Similarly to what was observed in the combined cohort, EXT1/EXT2-negative cases had statistically significantly higher values of SCr at diagnosis when compared with EXT1/EXT2-positive patients (0.80 vs. 0.70 mg/dL, *P*=0.010). The EXT1/EXT2 positive patients presented more frequently with proteinuria ≥ 3.5 g/day at diagnosis (57.5% vs. 35.3%, P*=*0.035). Kidney biopsies of EXT1/EXT2-negative patients showed a statistically significantly higher median of global glomerulosclerosis and IFTA (*P*<0.0001). At the end of the follow up, there were no differences between groups on the median SCr or proteinuria ≥3.5 g/day. During the course of the disease, patients who were EXT1/EXT2-negative evolved more frequently to ESKD (16.9% vs. 4.2%, *P*=0.036) but the mean time to ESKD was not statistically significantly different between groups (104 vs. 115 months, *P*=0.079, Figure 4B). Using univariable Cox regression, the predictive factors for the development of ESKD at 10 years were glomerulosclerosis ≥ 25% (HR 4.31; 95%CI, 1.440-12.876; *P*=0.009) and IFTA ≥ 25% (HR 3.91; 95%CI, 1.305 - 11.728; *P*=0.015) (Table 5).

### Class V LMN with class III/IV LN

Sixteen (25%) of 64 EXT1/EXT2-positive cases and 31 (32.3%) of 96 EXT1/EXT2-negative cases were class V LMN with proliferative features (class III or IV) (Table 4). At the time of diagnosis of LMN, there was no statistically significant difference between the two groups regarding SCr, hematuria, proteinuria, or presence of proteinuria ≥ 3.5 g/day. However, in EXT1/EXT2-negative patients, kidney biopsies showed a statistically significantly higher median of global glomerulosclerosis and IFTA (*P*=0.013 and *P*=0.003, respectively). At the end of the follow up, there were no differences between groups on the median SCr or proteinuria ≥3.5 g/day. EXT1/EXT2-positive patients with LN did not evolve to ESKD during the course of the disease (0.0% vs. 22.6%, *P*=0.039), whereas EXT1/EXT2-negative patients developed ESKD in a mean time of 93 months (*P*=0.031, Figure 4C). Using multivariable Cox regression, the predictive factors for the development of ESKD at 10 years were: male gender (HR 8.35; 95% CI, 1.11-62.6; *P*=0.039) and the SCr levels at the time of kidney biopsy (HR 1.53; 95%CI, 1.04-2.25; *P*=0.031) (Table 5).

### Summary

Exostosins (EXT1/EXT2) are glycosyltransferases that exist as heterodimeric co-polymerase complex responsible for synthesis of heparin sulfate chain in the glomerular basement membrane (Rops et al., Kidney Int., 86:932-942 (2014)). In this study of 374 patients of LMN, a subset (32.6%) of LMN were positive for EXT1/EXT2. EXT1/EXT2-positive LMN have less chronicity on kidney biopsy, have lower serum creatinine but a higher proportion of patients with proteinuria ≥3.5 g/day at presentation, and on follow-up develop markedly decreased incidence of ESKD compared to EXT1/EXT2-negative LMN. The difference in chronicity between the EXT1/EXT2-positive and EXT1/EXT2-negative LMN was noted in all groups, i.e. overall LMN with or without class III/IV LN, pure LMN, and LMN with class III/IV LN. Similarly, ESKD developed more frequently in EXT1/EXT2-negative LMN in all groups, i.e. overall LMN with or without class III/IV LN, pure LMN, and LMN with class III/IV LN, compared to EXT1/EXT2-positive LMN. These findings have significant bearing when considering the high burden of SLE in worldwide and in particular North America where the prevalence of SLE is 241/100,000 (Stojan et al., Curr. Opin. Rheumatol., 30:144-150 (2018)). Furthermore, the mortality rate is increased to ~2.6 fold in SLE, and the risk of mortality is the highest in patients with renal disease (Lee et al., Lupus, 25:727-734 (2016)). LN is the most common renal manifestation in SLE, almost 10% of the patients will develop ESKD, and 20% of the LN biopsies show LMN with or without concurrent class III/IV LN (Almaani et al., Clin. J. Am. Soc. Nephrol., 12:825-835 (2017); Alarcon, Reumatol. Clin., 7:3-6 (2011); Huong et al., Medicine (Baltimore), 78:148-166 (1999)).

The results provided herein demonstrate that EXT1/EXT2-positive LMN is a subgroup in LMN that is less likely to develop ESKD, and identification of this subgroup would be helpful to the management and prognosis of LMN. One question that arises is why do patients that are EXT1/EXT2-positive have better outcomes, despite higher proteinuria at presentation? Also, how does being positive for EXT1/EXT2 protect LMN from developing more progressive disease? Exostosins are present in the podocyte Golgi apparatus where they are responsible for the glycosylation of heparan sulfates that are eventually transported to the GBM (Busse-Wicher et al., Matrix Biology, 35:25-33 (2014); and Ahn et al., Nature Genetics, 11:137-143 (1995)). The exostosins have a short N-terminal cytoplasmic tail, a single transmembrane domain, a stem/stalk region, and a long globular catalytic C terminal domain (Duncan et al., J. Clin. Inv., 108:511-516 (2001); and McCormick et al., Proc. Natl. Acad. Sci. USA, 97:668-673 (2000)). EXT like other glycosyltransferases are secreted into the extracellular medium including the GBM in a truncated form (Paulson et al., J. Biol. Chem., 264:17615-17618 (1989)). It is hypothesize herein that in patients with EXT1/EXT2-positive LMN, the increased secretion of catalytic domain EXT1/EXT2 into the GBM results in increased synthesis of the heparan sulfates which in turn may offer protection from damaging events such as leukocyte infiltration, complement activation, cytokine production, etc (Figure 5). The EXT1/EXT2 along with heparan sulfates may in fact coat the immune-complexes and restrict the immune-deposits from generating an inflammatory response. Indeed, loss of heparan sulfates in GBM has been associated with development of proteinuria (van den Born et al., Kidney International, 43:454-463 (1993)), and dysregulation of the complement pathways by impairing complement regulation by complement factor H (van den Born et al., Kidney International, 43:454-463 (1993)). Furthermore, it was recently shown that heparan sulfates can act as clearance receptors for aberrant extracellular proteins, thus facilitating removing of the immune complexes and proteins involved in the immune response (Itakura et al., J. Cell Biol., 219:e201911126 (2020)). It is likely that EXT1/EXT2 is secreted by the podocytes as EXT1/EXT2 is present in only LMN in the setting of subepithelial deposits and not by mesangial cells or endothelial cells since EXT1/EXT2 is absent in class I/II/III/IV lupus nephritis where the deposits are mesangial or subendothelial in location.

The results provided herein indicate that cases of LN and LMN should be stained for EXT1/EXT2. The EXT1/EXT2-positive cases in LN may help detect an unknown component of LMN. In addition, it is reasonable to just stain for EXT1 instead of staining for both EXT1 and EXT2 since no single case showed isolated positivity and EXT1 appears brighter than EXT2. Finally, most cases of LMN were not re-biopsied. In this cohort, only four LMN cases were re-biopsied of which three were EXT1/EXT2-positive and one was EXT1/EXT2-negative. None of the EXT1/EXT2-positive LMN showed progression of the chronicity indexes while the single EXT1/EXT2-negative LMN case showed progression of chronicity indexes.

This was a retrospective study of LMN biopsies, and detailed follow-up was available in 160 (42.8%) patients of the 374 patients of LMN. Specific treatment details along with length of treatment were not available in many patients.

To summarize, 32.6% of LMN patients were positive for EXT1/EXT2. The extent of chronic changes including global glomerulosclerosis and tubular atrophy and interstitial fibrosis in EXT1/EXT2-positive LMN are less compared to EXT1/EXT2-negative LMN. EXT1/EXT2-positive LMN rarely develop ESKD compared to EXT1/EXT2-negative LMN.

## Claims

1. An *ex vivo* or *in vitro* method for identifying a mammal as having membranous nephropathy that is likely or unlikely to progress to end stage kidney disease, wherein said method comprises:
(a) determining if kidney tissue from said mammal expresses a polypeptide, wherein said polypeptide is an exostosin 1 (EXT1) polypeptide or an exostosin 2 (EXT2) polypeptide;
(b) classifying said mammal as having said membranous nephropathy that is unlikely to progress to said end stage kidney disease if said kidney tissue expresses said polypeptide; and
(c) classifying said mammal as having said membranous nephropathy that is likely to progress to said end stage kidney disease if said kidney tissue does not express said polypeptide.

2. The method of claim 1, wherein said method comprises classifying said mammal as having said membranous nephropathy that is unlikely to progress to said end stage kidney disease.

3. The method of claim 1 or claim 2, wherein said method comprises classifying said mammal as having said membranous nephropathy that is likely to progress to said end stage kidney disease.

4. The method of any one of claims 1-3, wherein said method comprises using immunohistochemistry to determine if said kidney tissue expresses said polypeptide.

5. The method of any one of claims 1-4, wherein said method comprises using laser microdissection and mass spectrometry to determine if said kidney tissue expresses said polypeptide.

6. The method of any one of claims 1-5, wherein said method comprises determining that said kidney tissue expresses said EXT1 polypeptide and said EXT2 polypeptide.

7. The method of any one of claims 1-6, wherein said method comprises determining that said kidney tissue does not express said EXT1 polypeptide and said EXT2 polypeptide.

8. An immunosuppressant for use in a method for treating a mammal having membranous nephropathy likely to progress to end stage kidney disease, wherein said method comprises:
(a) determining that kidney tissue from said mammal does not express a polypeptide, wherein said polypeptide is an exostosin 1 (EXT1) polypeptide or an exostosin 2 (EXT2) polypeptide; and
(b) administering said immunosuppressant to said mammal to reduce the rate of progression to said end stage kidney disease.

9. An immunosuppressant for use in a method for treating a mammal having membranous nephropathy likely to progress to end stage kidney disease, wherein said method comprises administering said immunosuppressant to a mammal identified as having kidney tissue that does not express a polypeptide, wherein said polypeptide is an exostosin 1 (EXT1) polypeptide or an exostosin 2 (EXT2) polypeptide.

10. The method of any one of claims 1-7 or the immunosuppressant for use of claim 8 or claim 9, wherein said mammal is a human.

11. The method of any one of claims 1-7 or the immunosuppressant for use of any one of claims 8-10, wherein said polypeptide is said EXT1 polypeptide.

12. The method of any one of claims 1-7 or the immunosuppressant for use of any one of claims 8-10, wherein said polypeptide is said EXT2 polypeptide.

13. The immunosuppressant for use of any one of claims 8-12, wherein said method comprises using immunohistochemistry to determine that said kidney tissue does not express said polypeptide, and/or wherein said method comprises using laser microdissection and mass spectrometry to determine that said kidney tissue does not express said polypeptide.

14. The immunosuppressant for use of any one of claims 8-13, wherein said method comprises determining that said kidney tissue does not express said EXT1 polypeptide and said EXT2 polypeptide.

15. The immunosuppressant for use of any one of claims 8-14, wherein the immunosuppressant is selected from the group consisting of corticosteroids, cyclosporine, mycophenolate mofetil and a B-cell reduction or depletion agent.

## Patentansprüche

1. Ein Ex-vivo- oder In-vitro-Verfahren zur Identifizierung eines Säugers als membranöse Nephropathie aufweisend, die wahrscheinlich oder wahrscheinlich nicht zu Nierenerkrankung im Endstadium fortschreiten wird, wobei das Verfahren Folgendes umfasst:
(a) Bestimmen, ob Nierengewebe von dem Säuger ein Polypeptid exprimiert, wobei das Polypeptid ein Exostosin-1(EXT1)-Polypeptid oder ein Exostosin-2(EXT2)-Polypeptid ist;
(b) Klassifizieren des Säugers als die membranöse Nephropathie, die wahrscheinlich nicht zu der Nierenerkrankung im Endstadium fortschreiten wird, aufweisend, wenn das Nierengewebe das Polypeptid exprimiert; und
(c) Klassifizieren des Säugers als die membranöse Nephropathie, die wahrscheinlich zu der Nierenerkrankung im Endstadium fortschreiten wird, aufweisend, wenn das Nierengewebe das Polypeptid nicht exprimiert.

2. Verfahren nach Anspruch 1, wobei das Verfahren das Klassifizieren des Säugers als die membranöse Nephropathie, die wahrscheinlich nicht zu der Nierenerkrankung im Endstadium fortschreiten wird, aufweisend umfasst.

3. Verfahren nach Anspruch 1 oder Anspruch 2, wobei das Verfahren das Klassifizieren des Säugers als die membranöse Nephropathie, die wahrscheinlich zu der Nierenerkrankung im Endstadium fortschreiten wird, aufweisend umfasst.

4. Verfahren nach einem der Ansprüche 1-3, wobei das Verfahren die Verwendung von Immunhistochemie umfasst, um zu bestimmen, ob das Nierengewebe das Polypeptid exprimiert.

5. Verfahren nach einem der Ansprüche 1-4, wobei das Verfahren die Verwendung von Lasermikrodissektion und Massenspektrometrie umfasst, um zu bestimmen, ob das Nierengewebe das Polypeptid exprimiert.

6. Verfahren nach einem der Ansprüche 1-5, wobei das Verfahren das Bestimmen umfasst, dass das Nierengewebe das EXT1-Polypeptid und das EXT2-Polypeptid exprimiert.

7. Verfahren nach einem der Ansprüche 1-6, wobei das Verfahren das Bestimmen umfasst, dass das Nierengewebe das EXT1-Polypeptid und das EXT2-Polypeptid nicht exprimiert.

8. Immunsuppressivum zur Verwendung in einem Verfahren zur Behandlung eines Säugers mit membranöser Nephropathie, die wahrscheinlich zu Nierenerkrankung im Endstadium fortschreiten wird, wobei das Verfahren Folgendes umfasst:
(a) Bestimmen, dass Nierengewebe von dem Säuger ein Polypeptid nicht exprimiert, wobei das Polypeptid ein Exostosin-1(EXT1)-Polypeptid oder ein Exostosin-2(EXT2)-Polypeptid ist; und
(b) Verabreichen des Immunsuppressivums an den Säuger, um die Rate des Fortschreitens zur Nierenerkrankung im Endstadium zu verringern.

9. Immunsuppressivum zur Verwendung in einem Verfahren zur Behandlung eines Säugers mit membranöser Nephropathie, die wahrscheinlich zu Nierenerkrankung im Endstadium fortschreiten wird, wobei das Verfahren das Verabreichen des Immunsuppressivums an einen Säuger umfasst, der identifiziert wurde als Nierengewebe aufweisend, das ein Polypeptid nicht exprimiert, wobei das Polypeptid ein Exostosin-1(EXT1)-Polypeptid oder ein Exostosin-2(EXT2)-Polypeptid ist.

10. Verfahren nach einem der Ansprüche 1-7 oder Immunsuppressivum zur Verwendung nach Anspruch 8 oder Anspruch 9, wobei es sich bei dem Säuger um einen Menschen handelt.

11. Verfahren nach einem der Ansprüche 1-7 oder Immunsuppressivum zur Verwendung nach einem der Ansprüche 8-10, wobei das Polypeptid das EXT1-Polypeptid ist.

12. Verfahren nach einem der Ansprüche 1-7 oder Immunsuppressivum zur Verwendung nach einem der Ansprüche 8-10, wobei das Polypeptid das EXT2-Polypeptid ist.

13. Immunsuppressivum zur Verwendung nach einem der Ansprüche 8-12, wobei das Verfahren die Verwendung von Immunhistochemie umfasst, um zu bestimmen, dass das Nierengewebe das Polypeptid nicht exprimiert, und/oder wobei das Verfahren die Verwendung von Lasermikrodissektion und Massenspektrometrie umfasst, um zu bestimmen, dass das Nierengewebe das Polypeptid nicht exprimiert.

14. Immunsuppressivum zur Verwendung nach einem der Ansprüche 8-13, wobei das Verfahren das Bestimmen umfasst, dass das Nierengewebe das EXT1-Polypeptid und das EXT2-Polypeptid nicht exprimiert.

15. Immunsuppressivum zur Verwendung nach einem der Ansprüche 8-14, wobei das Immunsuppressivum ausgewählt ist aus der Gruppe bestehend aus Corticosteroiden, Cyclosporin, Mycophenolatmofetil und einem B-Zell-Reduktions- oder -Depletionsmittel.

## Revendications

1. Procédé *ex vivo* ou *in vitro* pour identifier un mammifère comme ayant une néphropathie membranaire qui est susceptible ou peu susceptible d'évoluer vers une maladie rénale à un stade terminal, ledit procédé comprenant :
(a) la détermination si du tissu rénal dudit mammifère exprime un polypeptide, ledit polypeptide étant un polypeptide d'exostosine 1 (EXT1) ou un polypeptide d'exostosine 2 (EXT2) ;
(b) la classification dudit mammifère comme ayant ladite néphropathie membranaire qui est peu susceptible d'évoluer vers ladite maladie rénale à un stade terminal si ledit tissu rénal exprime ledit polypeptide ; et
(c) la classification dudit mammifère comme ayant ladite néphropathie membranaire qui est susceptible d'évoluer vers ladite maladie rénale à un stade terminal si ledit tissu rénal n'exprime pas ledit polypeptide.

2. Procédé selon la revendication 1, dans lequel ledit procédé comprend la classification dudit mammifère comme ayant ladite néphropathie membranaire qui est peu susceptible d'évoluer vers ladite maladie rénale à un stade terminal.

3. Procédé selon la revendication 1 ou la revendication 2, dans lequel ledit procédé comprend la classification dudit mammifère comme ayant ladite néphropathie membranaire qui est susceptible d'évoluer vers ladite maladie rénale à un stade terminal.

4. Procédé selon l'une quelconque des revendications 1 à 3, dans lequel ledit procédé comprend l'utilisation d'une immunohistochimie pour déterminer si ledit tissu rénal exprime ledit polypeptide.

5. Procédé selon l'une quelconque des revendications 1 à 4, dans lequel ledit procédé comprend l'utilisation d'une microdissection laser et d'une spectrométrie de masse pour déterminer si ledit tissu rénal exprime ledit polypeptide.

6. Procédé selon l'une quelconque des revendications 1 à 5, dans lequel ledit procédé comprend la détermination du fait que ledit tissu rénal exprime ledit polypeptide EXT1 et ledit polypeptide EXT2.

7. Procédé selon l'une quelconque des revendications 1 à 6, dans lequel ledit procédé comprend la détermination du fait que ledit tissu rénal n'exprime pas ledit polypeptide EXT1 et ledit polypeptide EXT2.

8. Immunosuppresseur destiné à être utilisé dans un procédé de traitement d'un mammifère ayant une néphropathie membranaire susceptible d'évoluer vers une maladie rénale à un stade terminal, ledit procédé comprenant :
(a) la détermination du fait qu'un tissu rénal dudit mammifère n'exprime pas un polypeptide, où ledit polypeptide est un polypeptide d'exostosine 1 (EXT1) ou un polypeptide d'exostosine 2 (EXT2) ; et
(b) l'administration dudit immunosuppresseur audit mammifère afin de réduire la vitesse d'évolution vers ladite maladie rénale à un stade terminal.

9. Immunosuppresseur destiné à être utilisé dans un procédé de traitement d'un mammifère ayant une néphropathie membranaire susceptible d'évoluer vers une maladie rénale à un stade terminal, dans lequel ledit procédé comprend l'administration dudit immunosuppresseur à un mammifère identifié comme ayant un tissu rénal qui n'exprime pas de polypeptide, dans lequel ledit polypeptide est un polypeptide exostosine 1 (EXT1) ou un polypeptide exostosine 2 (EXT2).

10. Procédé selon l'une quelconque des revendications 1 à 7 ou immunosuppresseur destiné à être utilisé selon la revendication 8 ou la revendication 9, dans lequel ledit mammifère est un humain.

11. Procédé selon l'une quelconque des revendications 1 à 7 ou immunosuppresseur destiné à être utilisé selon l'une quelconque des revendications 8 à 10, dans lequel ledit polypeptide est ledit polypeptide EXT1.

12. Procédé selon l'une quelconque des revendications 1 à 7 ou immunosuppresseur destiné à être utilisé selon l'une quelconque des revendications 8 à 10, dans lequel ledit polypeptide est ledit polypeptide EXT2.

13. Immunosuppresseur destiné à être utilisé selon l'une quelconque des revendications 8 à 12, dans lequel ledit procédé comprend l'utilisation d'une immunohistochimie pour déterminer le fait que ledit tissu rénal n'exprime pas ledit polypeptide, et/ou dans lequel ledit procédé comprend l'utilisation d'une microdissection laser et d'une spectrométrie de masse pour déterminer le fait que ledit tissu rénal n'exprime pas ledit polypeptide.

14. Immunosuppresseur destiné à être utilisé selon l'une quelconque des revendications 8 à 13, dans lequel ledit procédé comprend la détermination du fait que ledit tissu rénal n'exprime pas ledit polypeptide EXT1 et ledit polypeptide EXT2.

15. Immunosuppresseur destiné à être utilisé selon l'une quelconque des revendications 8 à 14, dans lequel l'immunosuppresseur est choisi dans le groupe constitué des corticostéroïdes, de la cyclosporine, du mycophénolate mofétil et d'un agent de réduction ou de déplétion de cellules B.
